# EUROPEAN PATENT SPECIFICATION

(11) **EP 1 994 132 B1**
(45) Date of publication and mention of the grant of the patent: **16.05.2012**
(21) Application number: 07758061.1
(22) Date of filing: 07.03.2007
(51) Int. Cl.: C11D 3/20, C11D 3/37, C11D 9/08, C11D 9/22, C11D 9/26, A61Q 19/10

(54) **STABLE SOAP BASED CLEANSING SYSTEM**
STABILES REINIGUNGSSYSTEM AUF SCHAUMSTOFFBASIS
SYSTÈME DE NETTOYAGE STABLE À BASE DE SAVON

(30) Priority: 08.03.2006 US 780327 P
(43) Date of publication of application: 26.11.2008
(73) Proprietor: Lubrizol Advanced Materials, Inc., Cleveland, OH 44141-3247 (US)
(72) Inventor: YAO, Ge, Hong Kong (CN); LIU, Xin, Hong Kong (CN); SHUSTER, Francine, Brecksville, OH 44141 (US); CASTNER, Julie, F., Strongsville, OH 44149 (US)
(74) Representative: von Kreisler Selting Werner
(86) International application number: PCT/US2007/063473
(87) International publication number: WO 2007/103968

(56) References cited:
- WO-A-01/76552
- WO-A-92/15665
- WO-A-96/17591
- WO-A-2005/030163
- WO-A-2006/012372
- WO-A-2006/100161
- GB-A- 2 297 975
- US-A1- 2005 201 965
- US-A1- 2006 073 996
- US-B2- 6 846 785

## Description

### FIELD OF THE INVENTION

The present invention relates to liquid skin cleansing compositions. More particularly, it relates to phase stable liquid skin cleansing compositions comprising a fatty acid soap, an acrylate based copolymer and an acidifying agent.

### BACKGROUND

Fatty acid liquid soap compositions are known in the art. These soaps have been widely employed for many years as effective general all purpose body cleansers. Fatty acid liquid soaps are formulated with a myriad of different ingredients to obtain the desired cleansing effect and the requisite physical property parameters so that they can be easily stored and dispensed in a convenient manner. Fatty acid liquid soaps must have the appropriate rheology characteristics to be flowable when dispensed from the product container but of a sufficient viscosity not run off of the skin when applied to the body. In addition, today's consumer is increasingly looking for additional benefits beyond the basic cleansing effects brought about by the traditional liquid soap product. Efforts are continually being made to make improvements in product function and aesthetics by incorporating various adjuncts into the formulation such as moisturizers, emollients, colorants, opacifiers, perfumes, antioxidants, antibacterial agents, and the like to name a few. It has also been increasingly popular to incorporate water insoluble moieties such as microcapsules, beads, and pearlescent agents into the liquid soap composition for delivery of actives to the skin and for product aesthetics.

In order to achieve the desired rheology profiles and to disperse the multitude of different ingredients within the soap composition, synthetic rheology modifying polymers and synthetic surfactants have been employed in an attempt to obtain a composition which is stable with respect to viscosity and visual phase homogeneity over a period of time and a wide range of temperatures. These parameters are particularly significant for liquid compositions wherein the large quantity of water in the formulation makes the establishment of a stable composition more difficult, particularly when substantially water insoluble adjuncts are dispersed in the formulation.

Japanese Patent Publication No. 2004-204153 (publication date July 22, 2004) discloses a cleansing composition containing (A) a fatty acid salt; (B) a sulfate ester surfactant; and (C) a copolymer obtained from the polymerization of at least one monomer selected from (meth)acrylic acid, maleic acid, and an alkyl (meth)acrylate.

Japanese Patent Publication No. H11-189785 (publication date July 13, 1999) concerns a cleansing agent containing (A) a fatty acid salt; (B) a polyhydric alcohol; and (C) and alkyl acrylate copolymer.

While these compositions are typically stable at ambient temperatures, the structuring of this type of soap formulation tends to collapse when exposed to elevated temperatures of about 40 to 50°C. Consequently, care must be taken when transporting and storing these products in warm and tropical regions of the world.

The use of synthetic surfactants in place of fatty acid soaps has been proposed to achieve a storage stable composition that is able to suspend water insoluble adjuncts. U.S. Patent No. 6,846,785 discloses a liquid soap composition containing a cleansing base formulated from an anionic surfactant and a chelating agent, vitamin microcapsules and a crosslinked, alkali-swellable acrylic emulsion polymer for uniform suspension of the microcapsules. However, synthetic surfactants are more expensive than fatty acid soaps and synthetic surfactants can be irritating to the eyes and skin. Moreover, when applied to the skin surfactants have a tendency to absorb moisture, swell and leave the skin feeling taunt or sticky.
WO 2006/012372 (Examples 1-3) discloses liquid hand soaps comprising Carbobol Aqua SF-1 (a crosslinked acrylic emulsion polymer), citric acid and further surfactants.
WO 92/15665 discloses liquid soaps comprising potassium fatty acid soap, polymeric thickener preferably an acrylate Acrysol, a non-crosslinked hydrophobically modified, alkali-soluble emulsion polymer that contains steareth-20 methacrylate.

Accordingly, there is a need for a fatty acid based liquid soap composition that retains good rheological profiles, structure and visual aesthetics across a wide temperature range while having the mildness and sensorial attributes that are superior to those of conventional liquid soaps.

### DESCRIPTION OF EXEMPLARY EMBODIMENTS

In accordance with a general embodiment of the present invention, the liquid cleansing soap comprises a fatty acid salt soap base, a crosslinked alkali-swellable acrylic emulsion polymer, and an acidifying agent. The composition is stabilized with respect to viscosity and to phase integrity with the crosslinked alkali-swellable acrylic emulsion polymer and subsequently back-acid treated with the acidifying agent to obtain compositions that are storage and phase stable over a wide temperature range.

Accordingly, the present invention relates to a liquid soap composition comprising:
A) 10% to 50% by weight, based on the weight of the total composition, of at least one C₈-C₂₂ fatty acid salt;
B) at least one crosslinked acrylic emulsion polymer polymerized from a monomer composition comprising:
   i) one or more unsaturated carboxylic acid containing monomers and salts thereof having a total of from 3 to 10 carbon atoms;
   ii) one or more vinyl monomers selected from the formulae:
      a) CH₂=CXY
         wherein X is H and Y is -COOR, -C₆H₄R', -CN, -CONH₂, -Cl, NC₄H₆O, -NH(CH₂)₃COOH, -NHCOCH₃, -CONHC(CH₃)₃, -CO-N(CH₃)₂,
         or X is CH₃ and Y is -COOR, -C₆H₄R', -CN or -CH=CH₂,
         or X is Cl and Y is Cl, and
         R is C₁-C₁₈ alkyl, or hydroxy C₂-C₁₈ alkyl,
         R' is H or C₁-C₁₈ alkyl;
      b) CH₂=CHOC(O)R¹
         wherein R¹ is C₁-C₁₈ alkyl; and
      c) CH₂=CH₂ or CH₂=CHCH₃; and
   iii) one or more polyunsaturated monomers;
C) an acidifying agent;
   wherein the pH of the liquid soap composition is from 7.8 to 9.8.

The invention further relates to a method for making the stable liquid soap composition as defined in claim 1 said method comprising:
A) providing a fatty acid soap composition comprising 10% to 50% by weight, based on the weight of the total composition, of at least one C₈ to C₂₂ fatty acid salt;
B) optionally increasing the pH of the fatty acid soap by adding a suitable base;
C) combining the fatty acid soap composition obtained in (A) or (B) with a crosslinked acrylic emulsion polymer polymerized from a monomer composition comprising:
   i) one or more unsaturated carboxylic acid containing monomers and salts thereof having a total of from 3 to 10 carbon atoms;
   ii) one or more vinyl monomers selected from the formulae:
      a) CH₂=CXY
         wherein X is H and Y is -COOR, -C₆H₄R¹, -CN, -CONH₂, -Cl, -NC₄H₆O, -NH(CH₂)₃COOH, -NHCOCH₃, -CONHC(CH₃)₃, -CO-N(CH₃)₂,
         or X is CH₃ and Y is -COOR, -C₆H₄R', -CN or -CH=CH₂,
         or X is Cl and Y is Cl, and
         R is C₁-C₁₈ alkyl, or hydroxy C₂-C₁₈ alkyl,
         R' is H or C₁-C₁₈ alkyl;
      b) CH₂=CHOC(O)R¹
         wherein R¹ is C₁-C₁₈ alkyl; and
      c) CH₂=CH₂ or CH₂=CHCH₃; and
   iii) one or more polyunsaturated monomers; and
D) reducing the pH of the composition obtained in (C) by at least 0.5 to 5 pH units below by the addition of an acidifying agent to obtain a liquid soap composition having a final pH of 7.8 to 9.8.

In one exemplary embodiment of the invention, the liquid soap composition comprises:
(a) from 10 to 50 wt. % of a fatty acid salt;
(b) from 0.1 to 10 wt. % of a crosslinked acrylic emulsion polymer;
(c) from 0.1 to 20 wt. % of an acidifying agent;
(d) an optional surfactant wherein the wt. ratio of fatty acid salt to surfactant ranges from 1:39 to 39:1;
(e) from 1 to 20 wt. % of an optional humectant;
(f) from 1 to 15 wt. % of an optional emollient; and
(g) the remainder water.

In addition to the foregoing ingredients, the composition can include other optional adjuncts conventionally used in liquid soaps. These include, for example, one or more preservatives, one or more viscosity adjusting agents, one or more skin conditioners, antibacterial agents, one or more antioxidants, one or more fragrances, one or more colorants, one or more chelating (sequestering) agents and one or more insoluble materials. These optional materials are described in more detail below.

The selection and the amounts of the forgoing ingredients will be dependent upon the desired liquid soap end product of the invention. For example, a hand soap, body wash, and facial cleanser can contain different ingredients as well as varying amounts of the same ingredient. The choice and amount of ingredients in formulated compositions of the invention will vary depending on the product and its function, as is well known to those skilled in the formulation arts. While overlapping weight ranges for the various ingredients that make up the liquid soap composition will be expressed for various embodiments of the invention, it should be readily apparent that the specific amount of each component in the composition will be selected from its disclosed range such that the desired amount of each component will be adjusted so that the sum of all components in the liquid soap composition totals 100 wt. %.
Preferred embodiments of the invention are apparent from the dependent claims.

### Fatty Acid Soap

In one aspect of the invention the liquid soap composition contains at least one the fatty acid soap containing from 8 to 22 carbon atoms. In another aspect of the invention the liquid soap composition contains at least one fatty acid soap containing from 12 to 18 carbon atoms. The fatty acids utilized in the soaps can be saturated and unsaturated and can be derived from synthetic sources, as well as from the hydrolysis of fats and natural oils. Exemplary saturated fatty acids include but are not limited to octanoic, decanoic, lauric, myristic, pentadecanoic, palmitic, margaric, steric, isostearic, nonadecanoic, arachidic, behenic, and the like, and mixtures thereof. Exemplary unsaturated fatty acids include but are not limited to myristoleic, palmitoleic, oleic, linoleic, linolenic, and the like, and mixtures thereof. The fatty acids can be derived from animal fat such as tallow or from vegetable oil such as coconut oil, red oil, palm kernel oil, palm oil, cottonseed oil, olive oil, soybean oil, peanut oil, corn oil, and mixtures thereof.

The soap can be prepared by a variety of well known means such as by the direct neutralization of a fatty acid or mixtures thereof or by the saponification of suitable fats and natural oils or mixtures thereof with a suitable base. Exemplary bases include potassium hydroxide, potassium carbonate, sodium hydroxide and triethanolamine. Generally, the fat or oil is heated until liquefied and a solution of aqueous base is added thereto. The pH of the system is adjusted by appropriate means, such as the addition of fatty acid or base, to be within the range of 8.5 to 14.0, in one aspect, and from 9.0 to 12.0, in another aspect of the invention, and from 9.5 to 11.0 in still another aspect of the invention. The upper limit of the pH range is established to ensure mildness toward the skin of the user and the lower limit serving to ensure neutralization or saponification of the fatty acid.

The amount of fatty acid soap that is employed in the liquid soap composition ranges from 10% to 50% by wt of the total composition in one aspect, and from 10% to 35% by wt. in another aspect of the invention.

In one aspect of the invention, the fatty acid soap is an alkali metal fatty acid soap. In another aspect of the invention the fatty acid soap is the potassium salt of a fatty acid.

### Crosslinked Acrylic Emulsion Polymer

The acrylic emulsion polymer of the present invention is polymerized from a monomer mixture comprising three polymerizable monomeric components. The first monomeric component is selected from one or more unsaturated carboxylic acid containing monomers and salts thereof having a total of from about 3 to about 10 carbon atoms. Examples of such monomers include but are not limited to acrylic acid, methacrylic acid, itaconic acid, fumaric acid, crotonic acid, maleic acid, or aconitic acid. Moreover, half esters of polyacids, such as maleic acid, fumaric acid, itaconic acid, or aconitic acid and the like with C₁ to C₄ alkanols can also be used, particularly if it is used in minor amounts in combination with acrylic acid or methacrylic acid. Salts of the foregoing monomers (e.g., sodium and potassium) can be employed.

The amounts of such carboxylic acid monomers is generally from 20% to 80% by wt. in one aspect, from 25% to 70% by wt. in another aspect and from 35% to 65% by wt in a further aspect, based upon the total wt. of the monomers.

The second monomeric component is selected from one or more vinyl monomers represented by the formulae:
1) CH₂=CXY,
   where X is H and Y is -C(O)OR, -C₆H₄R', -CN, -C(O)NH₂, -Cl, -NC₄H₆O, -NH(CH₂)₃C(O)OH, -NHC(O)CH₃, -C(O)NHC(CH₃)₃, -C(O)N(CH₃)₂,
   or X is CH₃ and Y is -C(O)OR, -C₆H₄R', -CN or -CH=CH₂;
   or X is Cl and Y is Cl, and
   R is C₁-C₁₈ alkyl, or hydroxy C₂-C₁₈ alkyl,
   R' is H or C₁-C₁₈ alkyl;
2) CH₂=CHOC(O)R¹,
   where R¹ is C₁-C₁₈ alkyl; or
3) CH₂=CH₂ or CH₂=CHCH₃.

Typical of such vinyl monomers or mixture of monomers are the various acrylate or hydroxy acrylate esters wherein the ester portion has from 1 to 10 carbon atoms such as methyl acrylate, ethyl acrylate, n-butyl acrylate, 2-ethylhexyl acrylate, 2-hydroxyethyl acrylate, or various vinyl acetates, or styrene, or vinyl chloride, or vinylidene chloride, or acrylonitrile, acrylamide, N,N,-dimethylacrylamide, t-butyl-acrylamide, and their methacrylate analogs.

The amount of such non-acid vinyl monomers is generally from 80% to 15% by wt. in one aspect, from 75% to 25% by wt. in another aspect, and from 65% to 35% by wt. in a further aspect based upon the total wt. of the monomers.

The third monomeric component forming the crosslinked acrylic emulsion polymer is one or more polyunsaturated crosslinking monomers. Monomeric unsaturated compounds carrying a reactive group that is capable of causing a formed copolymer to be crosslinked before, during, or after polymerization has taken place can also be used.

The polyunsaturated compound is utilized to generate either a partially or substantially-crosslinked three-dimensional polymeric network. By polyunsaturated is meant that the crosslinking monomer contains at least two polymerizable double bonds that are reactive with the foregoing unsaturated carboxylic acid containing monomers and the vinyl monomers. Examples of such polyunsaturated compounds are the polyalkenyl ethers of sucrose, or polyalcohols; diallylphthalates, divinyl benzene, allyl (meth)acrylate, ethylene glycol di(meth)acrylate, methylene bisacrylamide, trimethylolpropane tri(meth)acrylate, diallyl itaconate, diallyl fumarate, diallyl maleate, zinc (meth)acrylate, derivatives of castor oils or polyols made from ethylenically unsaturated carboxylic acid and the like, glycidyl methacrylate, N-methylol acylamide or N-alkoxymethylacrylamide, alkoxy being C₁ to C₁₈ alcohol; hydroxy(meth)acrylate or (meth)acrylate endcapped caprolactones.

For those skilled in the art of making unsaturated derivatives, a reaction scheme such as an esterification reaction of polyols made from ethylene oxide or propylene oxide or combinations thereof with unsaturated acid such as acrylic acid, methacrylic acid, or with unsaturated anhydride such as maleic anhydride, citraconic anhydride, itaconic anhydride, or an addition reaction with unsaturated isocyanate such as 3-isopropenyl-α-α-dimethylbenzene isocyanate (TMI), is also within the scope of the present invention.

As used herein, the term "(meth)acrylic" acid and "(meth)acrylate" are meant to include the corresponding methyl derivatives of acrylic acid and the corresponding alkyl acrylate. For example, "(meth)acrylic)" acid refers to acrylic acid and/or methacrylic acid and "(meth)acrylate" refers to alkyl acrylate and/or alkyl methacrylate.

The third component can be used in an amount from 0.01 to 5% by wt. in one aspect, from 0.03 to 3% by wt. in another aspect, and from 0.05 to 1% by wt. in a further aspect, based upon the total wt. of all of the monomer components.

Furthermore, the crosslinked copolymer acrylic emulsion is generally free of any moieties derived from associative monomers (i.e. copolymerizable surfactants). Generally free is defined as containing less than 1 % by wt. in one aspect, less than 0.5% by wt. in another aspect, and less than 0.2% by wt. in a further aspect of the invention.

The crosslinked acrylic emulsion polymer of the present invention can generally be prepared by emulsion polymerization techniques. The emulsion polymerization is generally carried out at a pH of from 2.5 to 5.0, with the at least three essentially ethylenically unsaturated components set forth above. None of these monomers are an associative monomer which is a copolymerizable surfactant capable of nonspecific hydrophobic association similar to those of conventional surfactants.

The crosslinked acrylic emulsion polymers of the present invention can be made in any conventional manner such as set forth in U.S. Patent No. 4,138,380, or U.S. Patent No. 4,110,291. Generally, one or more monomers of the above noted carboxylic acid monomers, vinyl monomers, and polyunsaturated monomers are added to a reaction vessel which contains water therein. Suitable amounts of conventional or typical emulsion polymerization surfactants such as sodium lauryl sulfate are added as well as emulsion type initiators, for example sodium or potassium persulfate, redox initiator, and the like. The reaction vessel can also contain a chain transfer agent. The temperature is then increased from 60°C to 100°C and polymerization commences. Optionally, during the reaction, additional monomers are added over a period of time. Upon completion of the addition of the monomers, polymerization is allowed to run to completion generally by adding additional initiator. Crosslinked Alkali-swellable acrylic emulsion polymers are disclosed in copending U.S. Patent Application No. 11/083,243 filed on March 17, 2005, now published as US 2005-0158268.

In one embodiment the crosslinked acrylic emulsion copolymer suitable for use in the liquid soap composition of the present invention is commercially available from Noveon, Inc., Cleveland, Ohio and is marketed under the tradename Carbopol^{®} Aqua SF-1. Carbopol Aqua SF-1 polymer is an alkali-swellable acrylic emulsion polymer composition containing approximately 30% by wt. polymer solids. The polymer is a branched to lightly crosslinked copolymer made from at least one pH sensitive first monomer selected from acrylic acid and/or methacrylic acid and at least one second monomer selected from the C₁ to C₅ alkyl esters of acrylic acid or methacrylic acid.

At an acidic pH the carboxyl functionality on the polymer backbone is in the protonated state and the polymer does not exhibit any viscosity building properties. However, after neutralization of the polymer by adding it to the alkaline soap composition which has a pH profile ranging from 8.5 to 14.0, the carboxyl groups on the polymer backbone ionize causing the polymer to swell and increase in viscosity.

The amount of the crosslinked acrylic emulsion copolymer employed in the liquid soap composition generally ranges from 0.1% to 10% by wt. of the total composition in one aspect, from 0.15% to 5% by wt. in another aspect, and in a further aspect from 0.3% to 4% of active (neat) polymer based upon the total wt. of the composition.

### Acidifying Agent

Surprisingly, it has been discovered that if the pH of the liquid soap composition is lowered subsequent to the addition of the crosslinked alkali-swellable acrylic emulsion copolymer rheology modifier, the viscosity and yield value of the composition generally remain unchanged or often actually increase. This formulating technique is referred to as "back-acid" thickening. The back-acid thickening technique can be used to further increase the viscosity and stability of compositions formulated in the alkaline pH range by actually reducing the pH of the composition.

In one aspect of the invention, the final target pH (subsequent to back-acid formulating) should be at least 0.5 to 5 pH units below that of the initial pH of the system. In another aspect the target pH is at least 0.75 to 5 pH units below the initial pH, and in still another aspect the target pH is at least 1 to 4 pH units below the initial pH of the soap composition.

An acidifying agent is added to reduce the pH of the composition. The acidifying agent can be an organic acid, such as citric acid, acetic acid, alpha-hydroxy acid, beta-hydroxy acid, salicylic acid, lactic acid, glycolic acid, natural fruit acids, or combinations thereof. In addition, inorganic acids, for example hydrochloric acid, nitric acid, sulfuric acid, sulfamic acid, phosphoric acid, and combinations thereof can be utilized to reduce the pH value of the composition. The amount of such acid is generally from 0.1 to 20%, in one aspect from 0.2% to 15% in another aspect, and from 0.25% to 10% by wt. in a further aspect, based upon the total wt. of the stabilized composition.

The desired pH to stabilize compositions of the present invention is obviously dependent on the specific viscosity and mildness parameters desired. According to the invention, the liquid soap compositions have a final pH range of 7.8 to 9.8. In another embodiment the final pH range of the liquid soap composition can range from 8.0 to 9.5, and in a further embodiment the pH can range from 8.2 to 9.2.

### Surfactant

The fatty acid soap compositions of the invention can include an optional surfactant selected from anionic, amphoteric, zwitterionic, nonionic, and combinations thereof.

The anionic surfactant can be any of the anionic surfactants known or previously used in the art of aqueous surfactant compositions. Suitable anionic surfactants include but are not limited to alkyl sulfates, alkyl ether sulfates, alkaryl sulfonates, alkyl succinates, alkyl sulfosuccinates, N-alkoyl sarcosinates, alkyl phosphates, alkyl ether phosphates, alkyl ether carboxylates, alkylamino acids, alkyl peptides, alkoyl taurates, carboxylic acids, acyl and alkyl glutamates, alkyl isethionates, and alpha-olefin sulfonates, especially their sodium, potassium, magnesium, ammonium and mono-, di- and triethanolamine salts. The alkyl groups generally contain from 8 to 18 carbon atoms and may be unsaturated. The alkyl ether sulfates, alkyl ether phosphates and alkyl ether carboxylates may contain from 1 to 10 ethylene oxide or propylene oxide units per molecule, and preferably contain 1 to 3 ethylene oxide units per molecule.

Examples of suitable anionic surfactants include sodium and ammonium lauryl ether sulfate (with 1, 2, and 3 moles of ethylene oxide), sodium, ammonium, and triethanolamine lauryl sulfate, disodium laureth sulfosuccinate, sodium cocoyl isethionate, sodium C₁₂₋₁₄ olefin sulfonate, sodium laureth-6 carboxylate, sodium C₁₂₋₁₅ pareth sulfate, sodium methyl cocoyl taurate, sodium dodecylbenzene sulfonate, sodium cocoyl sarcosinate, and triethanolamine monolauryl phosphate.

Amphoteric and zwitterionic surfactants are those compounds which have the capacity of behaving either as an acid or a base. These surfactants can be any of the surfactants known or previously used in the art of aqueous surfactant compositions. Suitable materials include but are not limited to alkyl betaines, alkyl amidopropyl betaines, alkyl sulphobetaines, alkyl glycinates, alkyl carboxyglycinates, alkyl amphopropionates, alkyl amidopropyl hydroxysultaines, acyl taurates and acyl glutamates wherein the alkyl and acyl groups have from 8 to 18 carbon atoms. Examples include cocamidopropyl betaine, sodium cocoamphoacetate, cocamidopropyl hydroxysultaine, and sodium cocamphopropionate.

The nonionic surfactant can be any of the nonionic surfactants known or previously used in the art of aqueous surfactant compositions. Suitable nonionic surfactants include but are not limited to aliphatic (C₆ to C₁₈) primary or secondary linear or branched chain acids, alcohols or phenols, alkyl ethoxylates, alkyl phenol alkoxylates (especially ethoxylates and mixed ethoxy/propoxy), block alkylene oxide condensate of alkyl phenols, alkylene oxide condensates of alkanols, ethylene oxide/propylene oxide block copolymers, semi-polar nonionics (e.g., amine oxides and phosphine oxides), as well as alkyl amine oxides. Other suitable nonionics include mono or di alkyl alkanolamides and alkyl polysaccharides, sorbitan fatty acid esters, polyoxyethylene sorbitan fatty acid esters, polyoxyethylene sorbitol esters, polyoxyethylene acids, and polyoxyethylene alcohols. Examples of suitable nonionic surfactants include coco mono or diethanolamide, coco diglucoside, alkyl polyglucoside, cocamidopropyl and lauramine oxide, polysorbate 20, ethoxylated linear alcohols, cetearyl alcohol, lanolin alcohol, stearic acid, glyceryl stearate, PEG-100 stearate, and oleth 20.

Other surfactants which can be utilized in the present invention are set forth in more detail in WO 99/21530, U.S. Patent No. 3.929.678. U.S. Patent No. 4,565,647, U.S. Patent No. 5,720,964, and U.S. Patent No. 5,858,948. Other suitable surfactants are described in McCutcheon's Emulsifiers and Detergents (North American and International Editions, by Schwartz, Perry and Berch).

In one embodiment of the invention, the amounts of surfactant can vary widely if present. The amounts which are often utilized generally range from 1% to 80% in one aspect, from 5% to 65% in another aspect, from 6% to 30% in a further aspect, and from 8% to 20% wt. based upon the total wt. of the composition in a still further aspect of the invention.

In other embodiment, the amount of surfactant utilized in the composition can be based on the amount of fatty acid soap present in the liquid cleansing composition, and can be expressed as a ratio of fatty acid soap to surfactant. In one aspect of the invention, the ratio of fatty acid soap:surfactant ranges from 39:1 to 1:39 (wt./wt.), in another aspect the soap:surfactant ratio ranges from 10:1 to 1 :10, and in a further aspect the soap:surfactant ratio ranges from 5:1 to 1:5.

### Humectant

The fatty acid liquid soap composition of the invention optionally includes at least one humectant. Suitable humectants include allantoin; pyrrolidonecarboxylic acid and its salts, hyaluronic acid and its salts, sorbic acid and its salts, urea, lysine, arginine, cystine, guanidine, and other amino acids, polyhydroxy alcohols such as glycerin, propylene glycol, hexylene glycol, hexanetriol, ethoxydiglycol, dimethicone copolyol, and sorbitol, and the esters thereof, polyethylene glycol, glycolic acid and glycolate salts (e.g. ammonium and quaternary alkyl ammonium), lactic acid and lactate salts (e.g. ammonium and quaternary alkyl ammonium), sugars and starches; sugar and starch derivatives (e.g. alkoxylated glucose); panthenols such as dl-panthenol; lactamide monoethanolamine; acetamide monoethanolamine; and the like, and mixtures thereof. In one embodiment the humectants include the C₃ to C₆ diols and triols, such as glycerin, propylene glycol, hexylene glycol, hexanetriol, and the like, and mixtures thereof. Such suitable humectants typically comprise
1 wt. % to 20 wt. % in one aspect of the invention, 2 wt. % to 10 wt. % in another aspect, and 3 wt. % to 5 wt. % in a further aspect based on the total wt. of the liquid soap composition.

### Emollient

The fatty acid liquid soap composition of the invention optionally includes at least one emollient. Suitable emollients include mineral oil; vegetable oil; hydrogenated vegetable oil, stearic acid; fatty alcohols such as cetyl alcohol, cetearyl alcohol, myristyl alcohol, behenyl alcohol, and lauryl alcohol, cetyl acetate in acetylated lanolin alcohol, benzoate esters such as C₁₂ to C₁₅ alkyl benzoates, isostearyl benzoate, dicaprylyl maleate, petrolatum, lanolin, coco butter, shea butter, beeswax and esters there of, ethoxylated fatty alcohol esters such as ceteareth-20, oleth-5, and ceteth-5, alkoxylated methyl glucose ethers such as gluceth-20, alkoxylated fatty acid esters such as polyethylene glycol 400 propoxylated monolaurate, avocado oil or glycerides, sesame oil or glycerides, safflower oil or glycerides, sunflower oil or glycerides, and other mono-, di-, and triglycerides of natural vegetable and botanical oils, such as, for example, caprylic triglyceride, capric triglyceride, caprylic/capric triglyceride, and caprylic/capric/lauric triglyceride, Guerbet esters such as G-20, G-36, G-38, and G-66 marketed by Noveon, Inc., botanical seed oils, volatile silicone oils, non-volatile emollients, and the like; and mixtures thereof. Suitable non-volatile emollients include fatty acid and fatty alcohol esters, highly branched hydrocarbons, and the like, and mixtures thereof. Such fatty acid and fatty alcohol esters include decyl oleate, butyl stearate, octyl stearate, myristyl myristate, octyldodecyl stearoylstearate, octylhydroxystearate, di-isopropyl adipate, isopropyl myristate, isopropyl palmitate, ethyl hexyl palmitate, isodecyl neopentanoate C₁₂ to C₁₅ alcohol benzoate, diethyl hexyl maleate, PPG-14 butyl ether and PPG-2 myristyl ether propionate, cetearyl octanoate, and the like, and mixtures thereof. Suitable highly branched hydrocarbons include isohexadecane and the like, and mixtures thereof. The emollients if present, alone or in combination, typically comprise 1 wt. % to 15 wt. % in one aspect, 2 wt. % to 10 wt. % in another aspect, and 3 wt. % to 5 wt. % in a further aspect, based on the total wt. of the liquid soap composition.

### Other Optional Components

The fatty acid liquid soap compositions of the invention can contain a variety of other conventional optional components suitable for rendering the cleansing compositions more desirable. Such optional components are well known to those skilled in the art of formulating liquid soap compositions. These include, for example, one or more alkaline and acidic pH adjusting agents, one or more preservatives, one or more viscosity adjusting agents, one or more skin conditioners, one or more antibacterial agents, one or more antioxidants, one or more fragrances, one or more colorants, botanical extracts, and one or more insoluble materials.

Non-limiting examples of alkaline pH adjusting agents include alkali metal hydroxides, such as sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol), and tetrakis(hydroxypropyl)ethylenediamine; and alkali metal salts of inorganic acids, such as sodium borate (borax), sodium phosphate, sodium pyrophosphate, and the like, and mixtures thereof. Acidic pH adjusting agents can be organic acids, including amino acids, and inorganic mineral acids. Non-limiting examples of acidic pH adjusting agents include acetic acid, citric acid, fumaric acid, glutamic acid, glycolic acid, hydrochloric acid, lactic acid, nitric acid, phosphoric acid, sodium bisulfate, sulfuric acid, tartaric acid, and the like, and mixtures thereof. The foregoing pH adjusting agents can be utilized to adjust the pH of the liquid soap composition to a desired pH value or range.

Suitable preservatives if present include polymethoxy bicyclic oxazolidine, methyl paraben, propyl paraben, ethyl paraben, butyl paraben, benzoic acid and the salts of benzoic acid, e.g., sodium benzoate, benzyltriazole, DMDM hydantoin (also known as 1,3-dimethyl-5,5-dimethyl hydantoin), imidazolidinyl urea, phenoxyethanol, phenoxyethylparaben, methylisothiazolinone, methylchloroisothiazolinone, benzoisothiazolinone, triclosan, sorbic acid, salicylic acid salts, and the like, and mixtures thereof. Such suitable preservatives typically comprise 0.01 wt. % to 1.5 wt. % of the total wt. of the personal care compositions of the present invention.

Suitable viscosity adjusting agents if present include organic and inorganic compounds, and combinations thereof. Examples of organic compounds include isopropyl alcohol, ethanol, sorbitol, propylene glycol, diethylene glycol, triethylene glycol, dimethyl ether, butylene glycol, and the like, and mixtures thereof. Examples of inorganic compounds include sodium chloride, sodium sulfate, potassium chloride, potassium nitrate, and mixtures thereof. Such suitable viscosity adjusters typically comprise 0.1 wt. % to 20 wt. % of the total wt. of the fatty acid liquid soap compositions of the present invention.

Skin conditioning polymers include quaternized guar gum, quaternized cellulosics, polyquaternium 4, polyquaternium 7, silicone quaternium-8 (dimethicone copolyol quaternized with an alkylamido dimethylamine, polyquaternium 10, polyquaternium 11, polyquaternium 39, polyquaternium 44, and the like, and mixtures thereof. Such suitable conditioning agents if present typically comprise 0.01 wt. % to 3 wt. % of the total wt. of the composition of the present invention.

Suitable chelating agents include EDTA (ethylene diamine tetraacetic acid) and salts thereof such as disodium EDTA, citric acid and salts thereof, cyclodextrins, and the like, and mixtures thereof. Such chelators if present typically comprise 0.001 wt. % to 3 wt. % of the total wt. of the liquid soap composition.

Suitable antioxidants if present include BHA and BHT and can be added at levels of 0.01 wt. % to 1.5 wt. % of the total wt. of the composition.

Insoluble materials include materials that impart pearlescent and other aesthetic visual, sensory and/or beneficial effects to the liquid soap composition. Some formulations are opacified by deliberately incorporating pearlescent materials therein to achieve a cosmetically attractive pearl-like appearance, known as pearlescence. Persons skilled in the art are aware of problems faced by formulators in consistently preparing a pearlescent formulation. A detailed discussion is found in the article "Opacifiers and pearling agents in shampoos" by Hunting, Cosmetic and Toiletries, Vol. 96, pages 65 to 78 (July 1981).

The pearlescent material includes titanium dioxide coated mica, iron oxide coated mica, ethylene glycol mono-stearate, ethylene glycol distearate, polyethylene glycol distearate, bismuth oxychloride coated mica, myristyl myristate, guanine, glitter (polyester or metallic), and mixtures thereof. Other pearlescent materials can be found in U.S. Patent No. 4,654,207 and U.S. Patent No. 5,019,376.

An improved or enhanced pearlescent appearance has been observed when back-acid formulating the compositions of the invention. Furthermore, the formulation technique prevents the sedimentation or precipitation of the pearlescent material thus significantly decreasing the "flattening out" of the pearlescent appearance. Additionally, it is believed that the polymeric rheology modifier serves to hold the pearlescent particles or platelets in their optimal configuration for maximum pearlescent appearance.

The amount of the pearlescent material can generally be used in amounts of from 0.05% to 10% and desirably from 0.15% to 3% by wt. based upon the total wt. of the composition.

In addition to the above generally insoluble compounds, numerous other optional substantially insoluble compounds which require stabilization can be utilized in the composition. Examples of such other insoluble compounds include titanium dioxide; pumice; calcium carbonate, talc, potato starch, tapioca starch, jojoba beads, polyethylene beads, walnut shells, loofah, apricot seeds; almond meal, corn meal, paraffin, oat bran/oat hulls, gelatin beads, alginate beads, stainless steel fibers, iron oxide pigments, air bubbles, mica coated iron oxides, kaolin clay, salicylic acid, zinc oxide, zeolite, styrofoam beads, phosphates, silica, and the like. Other generally insoluble compounds include teatree powder, microsponges, Confetti (a trademark of united guardian company), talc, beeswax, and the like. The amount of the various insoluble compounds requiring stabilization will vary depending upon its purpose, desired end result, and efficacy thereof. Hence amounts can vary widely, but frequently will be within a general range of from 0.1% to 20% by wt. based upon the total wt. of the soap composition.

A diluent such as water (often deionized) can be used and typically comprises 5 wt. % to 95 wt. % in one aspect, from 10 wt. % to 75 wt. % in another aspect, and from 15 wt. % to 50 wt. % in a further aspect of the invention of the total wt. of the fatty acid soap composition.

The following examples are presented for the purpose of illustrating the invention disclosed herein in greater detail. However, the examples are not to be construed as limiting the invention in any manner. It is to be appreciated that the forgoing formulations are set forth in an illustrative manner and that other liquid soap compositions and the formulation thereof are within the scope of the present invention. Unless otherwise specified all ingredient amounts are given by wt. % based on the weight of the composition.

### Methods Description

### Viscosity

The reported viscosity of each polymer containing composition was measured in milli-Pascal seconds (mPa·s), employing a Brookfield rotating spindle viscometer, (Brookfield, Model RVT) at about 20 revolutions per minute (rpm), at ambient room temperature of about 20 to 25°C. (hereafter referred to as Brookfield viscosity). Viscosity was measured on freshly prepared compositions (referred to as "initial viscosity", and re-measured after allowing the composition to age for at least about 24 hours at ambient room temperature (referred to as "24-hour viscosity"). Where only one viscosity value is shown, the viscosity value is the 24-hour viscosity, unless otherwise indicated.

### Yield Value

Yield Value, also referred to as Yield Stress, is herein defined as the initial resistance to flow under stress. It can be measured using a number of techniques, such as via the use of a constant stress rheometer or via extrapolation using a Brookfield viscometer. These techniques and the usefulness of the Yield Value measurement are further explained in Technical Data Sheet Number 244 available from Noveon, Inc.

### Stability

The various liquid soap compositions made using the substantially crosslinked alkali-swellable acrylate copolymer rheology modifier of the present invention are stable. The stability requirements for a particular composition will vary with its end marketplace application as well as the geography in which it is to be bought and sold. An acceptable "shelf life" is subsequently determined for each composition. This refers to the amount of time that a composition should be stable across its normal storage and handling conditions, measured between the times that the composition is produced and when it is finally sold for consumption.

To eliminate the need to conduct stability studies in excess of one year, stability testing is conducted at stressed conditions in order to predict the shelf life of a composition. Typically, accelerated testing is conducted at elevated static temperatures, usually 45 to 50°C. A composition should be stable for at least 2 weeks, desirably 1 month, preferably 2 or 3 months, and most preferably 4 or 5 months at 45°C. Additionally, freeze-thaw cycling is often employed wherein the composition is cycled between a freezing temperature, usually 0°C, and an ambient temperature, usually 20 to 25°C. A composition should pass a minimum of 1 freeze-thaw cycle, preferably 3 cycles, and most preferably 5 cycles.

Products or compositions made according to the present invention are considered stable if they meet one or more of the following criteria:
1. There is no phase separation, settling, or creaming of any material in the composition. The composition should remain completely homogenous throughout its bulk. Separation is herein defined as the visible existence of 2 or more distinct layers or phases of any component in the formulation, including but not limited to insoluble matter, soluble matter, oily substances and the like.
2. The viscosity of the composition does not significantly increase or decrease over time, generally less than 50%, preferably less than 35%, and most preferably less than 20%.
3. The pH of the composition does not increase or decrease more than two pH units, preferably not more than one unit, and most preferably not more than one-half unit.
4. The rheology and texture of the composition does not significantly change over time to that which is unacceptable.

Products or compositions made according to the present invention are considered unstable if they do not meet one or more of the above listed criteria.

### Example 1

A liquid soap composition is prepared by the back-acid formulation technique utilizing the following recipe.

| | Ingredient | Wt. % |
|---|---|---|
| | **Part A** | |
| 1 | potassium hydroxide (85% active) (neutralizer) | 6.3 |
| 2 | deionized water (diluent) | 15 |

| | **Part B** | |
|---|---|---|
| 3 | lauric acid (fatty acid) | 12.0 |
| 4 | myristic acid (fatty acid) | 4.0 |
| 5 | stearic acid (fatty acid) | 4.0 |
| 6 | Carbopol^{®} Aqua SF-1¹ rheology modifier | 6.0 |
| 7 | glycerin (humectant) | 5.0 |
| 8 | deionized water (diluent) | 47.65 |

| | **Part C** | |
|---|---|---|
| 9 | Neolone™ 950² (preservative) | 0.05 |
| 10 | citric acid (25%) (acidifying agent) | q.s. |

| | | |
|---|---|---|
| ¹acrylates copolymer emulsion (30% active), Noveon, Inc. (crosslinked copolymer of (meth)acrylic acid and C₁ to C₅ alkyl esters of acrylic acid) ²methylisothiazolinone, Rohm and Haas | | |

Part A is prepared by dissolving potassium hydroxide in deionized water and heating the composition to 80°C. Part B is separately prepared by adding glycerin and the rheology modifier to deionized (D.I.) water while mixing. The fatty acids (ingredient Nos. 3, 4, and 5) are added to the glycerin/rheology modifier/water mixture with agitation and the temperature is raised to 80°C. Once the fatty acids melt and are homogeneously mixed, the other Part B ingredients are added to the composition. Part A is added to Part B while the temperature is maintained at 80°C. The Part AB composition is mixed for 30 to 60 minutes. Upon attaining a homogeneous mixture, the Part AB composition is allowed to cool at ambient room temperature. Upon cooling to 40°C, ingredient No. 9 is added to the Part AB. The formulation is cooled under agitation to ambient room temperature. The viscosity and yield value is measured at the initial pH of the formulation. The pH is sequentially lowered by back-acid treating with citric acid. The viscosity and yield values following the sequential reduction of the pH values of the liquid soap composition are reported in the table below.

**Table 1**

| | pH | Viscosity (mPa·s) | Yield Value (dynes/cm²) |
|---|---|---|---|
| Initial | 10.35 | 1654 | 43 |
| Back-Acid pH | 9.40 | 1930 | 54 |
| | 9.10 | 3000 | 70 |
| | 8.95 | 8900 | 300 |
| | 8.76 | 22,800 | 1300 |
| | 8.35 | 44,500 | 3000 |
| | 7.70 | 15,250 | 1700 |
| | 7.40 | 12,900 | 1400 |
| | 7.20 | 8000 | 1400 |

### Example 2

A liquid soap/surfactant blend composition is prepared by the back-acid formulation technique utilizing the following recipe.

| | Ingredient | Wt. % |
|---|---|---|
| | **Part A** | |
| 1 | potassium hydroxide (85% active) (neutralizer) | 3.6 |
| 2 | deionized water (diluent) | 35 |

| | **Part B** | |
|---|---|---|
| 3 | lauric acid (fatty acid) | 7.2 |
| 4 | myristic acid (fatty acid) | 2.4 |
| 5 | stearic acid (fatty acid) | 2.4 |

| | **Part C** | |
|---|---|---|
| 6 | ethylene glycol distearate (pealescent agent) | 1.5 |

| | **Part D** | |
|---|---|---|
| 7 | Carbopol^{®} Aqua SF-1¹ rheology modifier | 4.0 |
| 8 | glycerin (humectant) | 3.0 |
| 9 | deionized water (diluent) | 12.8 |

| | **Part E** | |
|---|---|---|
| 10 | sodium laureth sulfate (2 mole ethoxylate, 28% solution) (surfactant) | 14.6 |
| 11 | cocamidopropyl betaine (30% solution) (surfactant) | 13.4 |
| 12 | Neolone™ 950² (preservative) | 0.1 |

| | | |
|---|---|---|
| ¹acrylates copolymer emulsion (30% active), Noveon, Inc. ²methylisothiazolinone, Rohm and Haas | | |

Part A is prepared by dissolving potassium hydroxide in deionized water and heated to 80°C. The ingredients of Part B are slowly heated with mixing until melted and then further heated until the temperature reaches 80°C. Parts A and B are combined with mixing. Part C is added to the combined Part AB composition which is maintained at 80°C and mixed until dissolved. While the combined Part ABC composition cools at ambient room temperature, the ingredients of Parts D and E are added in numerical order with thorough mixing. The viscosity of the liquid soap composition is adjusted with the addition of 10 g of sodium chloride. The initial pH, viscosity and yield values of the composition are measured. Following the measurement of the viscosity and yield value at the initial pH, the pH is sequentially increased with potassium hydroxide (85% solution) to a pH of 9.5 and then sequentially reduced by the back-acid technique with citric acid (25% solution) to a pH of 8.6. The initial viscosity and yield values as well as the viscosity and yield values following the sequential increase and reduction of the pH of the liquid soap composition are reported in the table below.

**Table 2**

| pH | Viscosity (mPa·s) | Yield Value (dynes/cm²) |
|---|---|---|
| 9.00 (initial) | 3900 | 16 |
| 9.90 | 1860 | 6 |
| 9.50 (max.) | 1200 | 4 |
| 9.24 | 2100 | 7 |
| 9.04 | 3400 | 11 |
| 8.94 | 5400 | 20 |
| 8.83 | 8120 | 32 |
| 8.72 | 11,480 | 50 |
| 8.60 (min.) | 22,800 | 116 |

### Examples 3 to 6

Liquid soap compositions are formulated from the ingredients set forth below utilizing the back-acid formulation technique.

| | Ingredients | Ex. 3 wt. % | Ex. 4 wt. % | Ex. 5 wt. % | Ex. 6 wt. % |
|---|---|---|---|---|---|
| | **PART A** | | | | |
| 1 | KOH (85%) (neutralizing agent) | 3.60 | 3.60 | 3.60 | 3.60 |
| 2 | deionized water (diluent) | 40.00 | 40.00 | 40.00 | 40.00 |

| | **PART B** | | | | |
|---|---|---|---|---|---|
| 3 | lauric acid | 4.80 | 4.80 | 4.80 | 4.80 |
| 4 | myristic acid | 2.40 | 2.40 | 2.40 | 2.40 |
| 5 | stearic acid | 4.80 | 4.80 | 4.80 | 4.80 |

| | **PART C** | | | | |
|---|---|---|---|---|---|
| 6 | Carbopol^{®} Aqua SF-1 rheology modifier | 5.00 | 6.00 | 7.00 | 8.00 |
| 7 | glycerin (humectant) | 3.00 | 3.00 | 3.00 | 3.00 |
| 8 | deionized water (diluent) | 8.30 | 7.30 | 6.30 | 5.30 |

| | **PART D** | | | | |
|---|---|---|---|---|---|
| 9 | sodium laureth sulfate (2 mole ethoxylate; 28% solution) (surfactant) | 14.60 | 14.60 | 14.60 | 14.60 |
| 10 | cocamidopropyl betaine (30% solution) (surfactant) | 13.40 | 13.40 | 13.40 | 13.40 |
| 11 | Neolone™ 950² (preservative) | 0.10 | 0.10 | 0.10 | 0.10 |

| | | | | | |
|---|---|---|---|---|---|
| ¹acrylates copolymer emulsion (30% active), Noveon, Inc. ²methylisothiazolinone, Rohm and Haas | | | | | |

Part A is prepared by dissolving potassium hydroxide in deionized water and heated to 80°C. The ingredients of Part B are slowly heated with mixing until melted and then further heated until the temperature reaches 80°C. Part A is combined with Part B and mixed for 30 to 60 minutes. The temperature of the mix is maintained at 80°C. Upon attaining a homogeneous mix, the Part AB composition is slowly allowed to cool. Part C is separately formulated by adding the rheology modifier and glycerin to D.I. water with mixing. When the Part AB composition reaches a temperature range of between 60 to 70°C, Part C is added thereto and mixed. The Part D ingredients are then added to the Part ABC composition in numerical order. The initial pH, viscosity and yield values of the composition are measured. The pH of each formulation is increased once with potassium hydroxide (85% solution) and then reduced once by the back-acid technique with citric acid (25% solution). The viscosity and yield data at each pH value is set forth in the table below.

**Table 3**

| Ex. No. | pH | Viscosity (mPa·s) | Yield Value (dynes/cm²) |
|---|---|---|---|
| 3 | 9.64 | 2070 | 22 |
| | 12.9 | 1070 | 9 |
| | 9.28 | 8000 | 30 |
| 4 | 9.46 | 5400 | 41 |
| | 12.30 | 2300 | 32 |
| | 9.33 | 13,400 | 70 |
| 5 | 9.40 | 13,400 | 100 |
| | 11.07 | 4170 | 70 |
| | 9.48 | 11,500 | 90 |
| 6 | 9.30 | 26,000 | 230 |
| | 10.30 | 7230 | 110 |
| | 9.60 | 14,320 | 130 |

### Example 7

A liquid soap composition is prepared by the back-acid formulation technique utilizing the following recipe.

| | Ingredient | Wt. % |
|---|---|---|
| | **Part A** | |
| 1 | potassium hydroxide (91.5% active) (neutralizer) | 3.45 |
| 2 | deionized water (diluent) | 15.00 |

| | **Part B** | |
|---|---|---|
| 3 | lauric acid (fatty acid) | 6.00 |
| 4 | myristic acid (fatty acid) | 2.00 |
| 5 | Isostearic acid (fatty acid) | 4.99 |
| 6 | Carbopol^{®} Aqua SF-1¹ rheology modifier | 4.00 |
| 7 | glycerin (humectant) | 6.00 |
| 8 | deionized water (diluent) | 22.95 |

| | **Part C** | |
|---|---|---|
| 9 | ethylene glycol distearate (pealescent agent) | 1.50 |
| 10 | sodium laureth sulfate (2 mole ethoxylate; 28% solution) (surfactant) | 20.00 |
| 11 | cocamidopropyl betaine (30% solution) (surfactant) | 13.00 |
| 12 | silicone quaternium-8 (conditioner) | 0.65 |
| 13 | gluceth-20 (humectant) | 0.30 |
| 14 | fragrance | 0.10 |
| 15 | Neolone™ 950² (preservative) | 0.05 |
| 16 | citric acid (25%) (acidifying agent) | 1.00 |

| | | |
|---|---|---|
| ¹acrylates copolymer emulsion (30% active), Noveon, Inc. ²methylisothiazolinone, Rohm and Haas | | |

Part A is prepared by dissolving potassium hydroxide in deionized water and heated to 80°C. The ingredients of Part B are slowly heated with mixing until melted and then further heated until the temperature reaches 80°C. Part A is combined with Part B and mixed for 30 to 60 minutes. The temperature of the mix is maintained at 80°C. Upon attaining a homogeneous mix, the Part AB composition is slowly allowed to cool. When the Part AB composition reaches a temperature range of between 60 to 70°C, ingredient No. 9 is added and mixed until uniform. Ingredient Nos. 10 and 11 are added in order and mixed. When the composition reaches 40°C, ingredient Nos. 12 to 15 are added in numerical order to the composition and mixed. The composition is back-acid treated with citric acid to obtain the liquid soap product.

### Properties

| | |
|---|---|
| Appearance: | pearlized viscous liquid |
| pH: | 8.80 - 9.00 |
| Viscosity (mPa·s): | 5000-8000 |
| Stability: | Passed 2 months @ 45°C |
| | Passed 5 freeze/thaw cycles |

## Claims

1. A liquid soap composition comprising:
A) 10% to 50% by weight, based on the weight of the total composition, of at least one C₈-C₂₂ fatty acid salt;
B) at least one crosslinked acrylic emulsion polymer polymerized from a monomer composition comprising:
i) one or more unsaturated carboxylic acid containing monomers and salts thereof having a total of from 3 to 10 carbon atoms;
ii) one or more vinyl monomers selected from the formulae:
a) CH₂=CXY
wherein X is H and Y is -COOR, -C₆H₄R', -CN, -CONH₂, -Cl, NC₄H₆O, -NH(CH₂)₃COOH, -NHCOCH₃, -CONHC(CH₃)₃, -CO-N(CH₃)₂,
or X is CH₃ and Y is -COOR, -C₆H₄R', -CN or -CH=CH₂,
or X is Cl and Y is Cl, and
R is C₁-C₁₈ alkyl, or hydroxy C₂-C₁₈ alkyl,
R' is H or C₁-C₁₈ alkyl;
b) CH₂=CHOC(O)R¹
wherein R¹ is C₁-C₁₈ alkyl; and
c) CH₂=CH₂ or CH₂=CHCH₃; and
iii) one or more polyunsaturated monomers;
C) an acidifying agent;
wherein the pH of the liquid soap composition is from 7.8 to 9.8.

2. A liquid soap composition of claim 1 further comprising:
D) an optional surfactant selected from an anionic surfactant, a cationic surfactant, a nonionic surfactant, amphoteric surfactant, and mixtures thereof.

3. A liquid soap composition of claim 1, wherein said fatty acid salt is selected from the alkali metal and/or ethanolamine salt of a C₈ to C₂₂ fatty acid.

4. A liquid soap composition of claim 3, wherein said fatty acid salt is derived from saponified animal fat, saponified vegetable oil, and mixtures thereof.

5. A liquid soap composition of claim 3, wherein said fatty acid salt is derived from a base neutralized fatty acid.

6. A liquid soap composition of claim 1, wherein said unsaturated carboxylic acid containing monomer is selected from (meth)acrylic acid, itaconic acid, fumaric acid, crotonic acid, maleic acid, aconitic acid, half esters of polyacids with C₁ to C₄ alkanols, and mixtures thereof.

7. A liquid soap composition of claim 1, wherein said crosslinked acrylic emulsion polymer is polymerized from (meth)acrylic acid and a C₁ to C₅ alkyl (meth)acrylate.

8. A liquid soap composition of claim 1, wherein said acidifying agent is selected from an organic acid, an inorganic acid, and mixtures thereof.

9. A liquid soap composition of claim 7, wherein said acidifying agent is selected from citric acid, acetic acid, alpha-hydroxy acid, beta-hydroxy acid, salicylic acid, lactic acid, glycolic acid, natural fruit acids, hydrochloric acid, nitric acid, sulfuric acid, sulfamic acid, phosphoric acid, and combinations thereof.

10. A liquid soap composition of claim 1 further comprising an optional component selected from one or more of a pH adjusting agent, a preservative, a viscosity adjusting agent, a skin conditioner, an antibacterial agent, an antioxidant, a fragrance, a colorant, a chelating agent, a humectant, an emollient, an insoluble material, a pearlescent material, a diluent, and combinations thereof.

11. A liquid soap composition of claim 9, wherein said composition comprises:
A) from 10 to 50 wt. % of at least one fatty acid salt;
B) from 0.1 to 10 wt. % of a crosslinked acrylic emulsion polymer;
C) from 0.1 to 20 wt. % of an acidifying agent;
D) a surfactant wherein the wt. ratio of said fatty acid salt to said surfactant ranges from 1:39 to 39:1.
E) from 1 to 20 wt. % of an optional humectant; and
F) from 1 to 15 wt. % of an optional emollient.

12. A method for making the stable liquid soap composition as defined in claim 1 said method comprising:
A) providing a fatty acid soap composition comprising 10% to 50% by weight, based on the weight of the total composition, of at least one C₈ to C₂₂ fatty acid salt;
B) optionally increasing the pH of the fatty acid soap by adding a suitable base;
C) combining the fatty acid soap composition obtained in (A) or (B) with a crosslinked acrylic emulsion polymer polymerized from a monomer composition comprising:
i) one or more unsaturated carboxylic acid containing monomers and salts thereof having a total of from 3 to 10 carbon atoms;
ii) one or more vinyl monomers selected from the formulae:
a) CH₂=CXY
wherein X is H and Y is -COOR, -C₆H₄R¹, -CN, -CONH₂, -Cl, -NC₄H₆O, -NH(CH₂)₃COOH, -NHCOCH₃, -CONHC(CH₃)₃, -CO-N(CH₃)₂,
or X is CH₃ and Y is -COOR, -C₆H₄R', -CN or -CH=CH₂,
or X is Cl and Y is Cl, and
R is C₁-C₁₈ alkyl, or hydroxy C₂-C₁₈ alkyl,
R' is H or C₁-C₁₈ alkyl;
b) CH₂=CHOC(O)R¹
wherein R¹ is C₁-C₁₈ alkyl; and
c) CH₂=CH₂ or CH₂=CHCH₃; and
iii) one or more polyunsaturated monomers; and
D) reducing the pH of the composition obtained in (C) by at least 0.5 to 5 pH units below by the addition of an acidifying agent to obtain a liquid soap composition having a final pH of 7.8 to 9.8.

13. A method of claim 12, wherein said unsaturated carboxylic acid containing monomer is selected from (meth)acrylic acid, itaconic acid, fumaric acid, crotonic acid, maleic acid, aconitic acid, half esters of polyacids with C₁ to C₄ alkanols, and mixtures thereof.

14. A method of claim 12, wherein said crosslinked acrylic emulsion polymer is polymerized from (meth)acrylic acid and a C₁ to C₅ alkyl (meth)acrylate.

15. A method claim 12, wherein said acidifying agent is selected from an organic acid, an inorganic acid, and mixtures thereof.

16. A method of claim 12, wherein said acidifying agent is selected from citric acid, acetic acid, alpha-hydroxy acid, beta-hydroxy acid, salicylic acid, lactic acid, glycolic acid, natural fruit acids, hydrochloric acid, nitric acid, sulfuric acid, sulfamic acid, phosphoric acid, and combinations thereof.

## Patentansprüche

1. Flüssigseifenzusammensetzung, umfassend:
A) 10 bis 50 Gew.-%, bezogen auf das Gewicht der gesamten Zusammensetzung, wenigstens eines C₈-C₂₂-Fettsäuresalzes;
B) wenigstens ein vernetztes Acrylemulsionspolymer, das ausgehend von einer Monomerzusammensetzung polymerisiert ist, die Folgendes umfasst:
i) ein oder mehrere ungesättigte carbonsäurehaltige Monomere und Salze davon mit insgesamt 3 bis 10 Kohlenstoffatomen;
ii) ein oder mehrere Vinylmonomere, die aus den folgenden Formeln ausgewählt sind:
a) CH₂=CXY,
wobei X = H ist und Y = -COOR, -C₆H₄R', -CN, -CONH₂, -Cl, NC₄H₆O, -NH(CH₂)₃COOH, -NHCOCH₃, -CONHC(CH₃)₃, -CO-N(CH₃)₂ ist
oder X = CH₃ ist und Y = -COOR, -C₆H₄R', -CN oder -CH=CH₂ ist
oder X = Cl ist und Y = Cl ist und
R = C₁-C₁₈-Alkyl oder Hydroxy-C₂-C₁₈-alkyl ist,
R' = H oder C₁-C₁₈-Alkyl ist;
b) CH₂=CHOC(O)R¹,
wobei R¹ = C₁-C₁₈-Alkyl ist; und
c) CH₂=CH₂ oder CH₂=CHCH₃; und
iii) ein oder mehrere mehrfach ungesättigte Monomere;
C) ein Säuerungsmittel;
wobei der pH-Wert der Flüssigseifenzusammensetzung 7,8 bis 9,8 beträgt.

2. Flüssigseifenzusammensetzung gemäß Anspruch 1, weiterhin umfassend:
D) ein optionales Tensid, das aus einem anionischen Tensid, einem kationischen Tensid, einem nichtionischen Tensid, einem amphoteren Tensid und Gemischen davon ausgewählt ist.

3. Flüssigseifenzusammensetzung gemäß Anspruch 1, wobei das Fettsäuresalz aus dem Alkalimetall- und/oder Ethanolaminsalz einer C₈- bis C₂₂-Fettsäure ausgewählt ist.

4. Flüssigseifenzusammensetzung gemäß Anspruch 3, wobei das Fettsäuresalz von verseiftem tierischen Fett, verseiftem Pflanzenöl und Gemischen davon abgeleitet ist.

5. Flüssigseifenzusammensetzung gemäß Anspruch 3, wobei das Fettsäuresalz von einer basenneutralisierten Fettsäure abgeleitet ist.

6. Flüssigseifenzusammensetzung gemäß Anspruch 1, wobei das ungesättigte carbonsäurehaltige Monomer aus (Meth)acrylsäure, Itaconsäure, Fumarsäure, Crotonsäure, Maleinsäure, Aconitinsäure, Halbestern von Polysäuren mit C₁- bis C₄-Alkanolen und Gemischen davon ausgewählt ist.

7. Flüssigseifenzusammensetzung gemäß Anspruch 1, wobei das vernetzte Acrylemulsionspolymer ausgehend von (Meth)acrylsäure und einem C₁-bis C₅-Alkyl(meth)acrylat polymerisiert ist.

8. Flüssigseifenzusammensetzung gemäß Anspruch 1, wobei das Säuerungsmittel aus einer organischen Säure, einer anorganischen Säure und Gemischen davon ausgewählt ist.

9. Flüssigseifenzusammensetzung gemäß Anspruch 7, wobei das Säuerungsmittel aus Zitronensäure, Essigsäure, alpha-Hydroxysäure, beta-Hydroxysäure, Salicylsäure, Milchsäure, Glycolsäure, natürlichen Fruchtsäuren, Chlorwasserstoffsäure, Salpetersäure, Schwefelsäure, Amidosulfonsäure, Phosphorsäure und Kombinationen davon ausgewählt ist.

10. Flüssigseifenzusammensetzung gemäß Anspruch 1, weiterhin umfassend eine optionale Komponente, die aus einer oder mehreren der Komponenten pH-Regulator, Konservierungsmittel, Viskositätsmodifikator, Hautpflegemittel, antibakterielles Mittel, Antioxidans, Duftstoff, Färbemittel, Chelatisierungsmittel, Feuchthaltemittel, Emolliens, unlösliches Material, Perlglanzmaterial, Verdünnungsmittel und Kombinationen davon ausgewählt ist.

11. Flüssigseifenzusammensetzung gemäß Anspruch 9, wobei die Zusammensetzung Folgendes umfasst:
A) 10 bis 50 Gew.-% wenigstens eines Fettsäuresalzes;
B) 0,1 bis 10 Gew.-% eines vernetzten Acrylemulsionspolymers;
C) 0,1 bis 20 Gew.-% eines Säuerungsmittels;
D) ein Tensid, wobei das Gewichtsverhältnis des Fettsäuresalzes zu dem Tensid im Bereich von 1:39 bis 39:1 liegt;
E) 1 bis 20 Gew.-% eines optionalen Feuchthaltemittels; und
F) 1 bis 15 Gew.-% eines optionalen Emolliens.

12. Verfahren zur Herstellung der stabilen Flüssigseifenzusammensetzung, wie sie in Anspruch 1 definiert ist, wobei das Verfahren Folgendes umfasst:
A) Bereitstellen einer Fettsäureseifenzusammensetzung, die 10 bis 50 Gew.-%, bezogen auf das Gewicht der gesamten Zusammensetzung, wenigstens eines C₈-C₂₂-Fettsäuresalzes umfasst;
B) gegebenenfalls Erhöhen des pH-Werts der Fettsäureseife durch Hinzufügen einer geeigneten Base;
C) Kombinieren der in (A) oder (B) erhaltenen Fettsäureseifenzusammensetzung mit einem vernetzten Acrylemulsionspolymer, das ausgehend von einer Monomerzusammensetzung polymerisiert ist, die Folgendes umfasst:
i) ein oder mehrere ungesättigte carbonsäurehaltige Monomere und Salze davon mit insgesamt 3 bis 10 Kohlenstoffatomen;
ii) ein oder mehrere Vinylmonomere, die aus den folgenden Formeln ausgewählt sind:
a) CH₂=CXY,
wobei X = H ist und Y = -COOR, -C₆H₄R¹, -CN, -CONH₂, -Cl, NC₄H₆O, -NH(CH₂)₃COOH, -NHCOCH₃, -CONHC(CH₃)₃, -CO-N(CH₃)₂ ist
oder X = CH₃ ist und Y = -COOR, -C₆H₄R', -CN oder -CH=CH₂ ist
oder X = Cl ist und Y = Cl ist und
R = C₁-C₁₈-Alkyl oder Hydroxy-C₂-C₁₈-alkyl ist,
R' = H oder C₁-C₁₈-Alkyl ist;
b) CH₂=CHOC(O)R¹,
wobei R¹ = C₁-C₁₈-Alkyl ist; und
c) CH₂=CH₂ oder CH₂=CHCH₃; und
iii) ein oder mehrere mehrfach ungesättigte Monomere; und
D) Reduzieren des pH-Werts der in (C) erhaltenen Zusammensetzung um wenigstens 0,5 bis 5 pH-Einheiten durch Zugabe eines Säuerungsmittels, wobei man eine Flüssigseifenzusammensetzung mit einem endgültigen pH-Wert von 7,8 bis 9,8 erhält.

13. Verfahren gemäß Anspruch 12, wobei das ungesättigte carbonsäurehaltige Monomer aus (Meth)acrylsäure, Itaconsäure, Fumarsäure, Crotonsäure, Maleinsäure, Aconitinsäure, Halbestern von Polysäuren mit C₁- bis C₄-Alkanolen und Gemischen davon ausgewählt ist.

14. Verfahren gemäß Anspruch 12, wobei das vernetzte Acrylemulsionspolymer ausgehend von (Meth)acrylsäure und einem C₁- bis C₅-Alkyl(meth)-acrylat polymerisiert ist.

15. Verfahren gemäß Anspruch 12, wobei das Säuerungsmittel aus einer organischen Säure, einer anorganischen Säure und Gemischen davon ausgewählt ist.

16. Verfahren gemäß Anspruch 12, wobei das Säuerungsmittel aus Zitronensäure, Essigsäure, alpha-Hydroxysäure, beta-Hydroxysäure, Salicylsäure, Milchsäure, Glycolsäure, natürlichen Fruchtsäuren, Chlorwasserstoffsäure, Salpetersäure, Schwefelsäure, Amidosulfonsäure, Phosphorsäure und Kombinationen davon ausgewählt ist.

## Revendications

1. Composition de savon liquide comprenant :
A) 10 % à 50 % en poids sur la base du poids de la composition totale, d'au moins un sel d'acide gras en C₈ à C₂₂ ;
B) au moins un polymère d'émulsion acrylique réticulé polymérisé à partir d'une composition demonomère comprenant :
- i) un ou plusieurs monomères insaturés contenant un acide carboxylique et des sels de ceux-ci ayant au total de 3 à 10 atomes de carbone ;
- ii) un ou plusieurs monomères vinyliques choisis parmi les formules :
-- a) CH₂=CXY
dans laquelle X est H et Y est -COOR, -C₆H₄R', -CN, -CONH₂, -Cl, NC₄H₆O, -NH(CH₂)₃COOH, -NHCOCH₃, -CONHC(CH₃)₃, -CO-N(CH₃)₂,
ou X est CH₃ et Y est -COOR, -C₆H₄R', -CN ou -CH=CH₂,
ou X est Cl et Y est Cl, et
R est un groupe alkyle en C₁ à C₁₈ ou hydroxy-alkyle en C₂ à C₁₈,
R' est H ou un groupe alkyle en C₁ à C₁₈ ;
-- b) CH₂=CHOC(O)R¹
dans laquelle R¹ est un groupe alkyle en C₁ à C₁₈ ; et
-- c) CH₂=CH₂ ou CH₂=CHCN₃ ; et
- iii) un ou plusieurs monomères polyinsaturés ;
C) un agent acidifiant ;
dans laquelle le pH de la composition de savon liquide est de 7,8 à 9,8.

2. Composition de savon liquide selon la revendication 1, comprenant en outre :
D) un tensioactif optionnel choisi parmi un tensioactif anionique, un tensioactif cationique, un tensioactif non ionique, un tensioactif amphotère et leurs mélanges.

3. Composition de savon liquide selon la revendication 1, dans laquelle ledit sel d'acide gras est choisi parmi le sel de métal alcalin et/ou éthanolamine d'un acide gras en C₈ à C₂₂.

4. Composition de savon liquide selon la revendication 3, dans laquelle ledit sel d'acide gras est dérivé de graisse animale saponifiée, d'huile végétale saponifiée et leurs mélanges.

5. Composition de savon liquide selon la revendication 3, dans laquelle ledit sel d'acide gras est dérivé d'un acide gras neutralisé par une base.

6. Composition de savon liquide selon la revendication 1, dans laquelle ledit monomère contenant un acide carboxylique insaturé est choisi parmi l'acide (méth)acrylique, l'acide itaconique, l'acide fumarique, l'acide crotonique, l'acide maléique, l'acide aconitique, les semi-esters de polyacides avec des alcools en C₁ à C₄, et leurs mélanges.

7. Composition de savon liquide selon la revendication 1, dans laquelle ledit polymère d'émulsion acrylique réticulé est polymérisé à partir de l'acide (méth)acrylique et un alkyl-(méth)acrylate en C₁ à C₅.

8. Composition de savon liquide selon la revendication 1, dans laquelle ledit agent acidifiant est choisi parmi un acide organique, un acide inorganique et leurs mélanges.

9. Composition de savon liquide selon la revendication 7, dans laquelle ledit agent acidifiant est choisi parmi l'acide citrique, l'acide acétique, un acide alpha-hydroxy, un acide bêta-hydroxy, l'acide salicylique, l'acide lactique, l'acide glycolique, les acides de fruits naturels, l'acide chlorhydrique, l'acide nitrique, l'acide sulfurique, l'acide sulfamique, l'acide phosphorique et leurs combinaisons.

10. Composition de savon liquide selon la revendication 1, comprenant un composant optionnel choisi parmi un ou plusieurs agent d'ajustement du pH, conservateur, agent d'ajustement de la viscosité, conditionneur cutané, agent antibactérien, antioxydant, parfum, colorant, agent chélateur, humectant, émollient, matériau insoluble, matériau opalescent, diluant et leurs combinaisons.

11. Composition de savon liquide selon la revendication 9, dans laquelle ladite composition comprend :
A) de 10 à 50 % en poids d'au moins un sel d'acide gras ;
B) de 0,1 à 10 % en poids d'un polymère d'émulsion acrylique réticulé ;
C) de 0,1 à 20 % en poids d'un agent acidifiant ;
D) un tensioactif dans lequel le rapport en poids dudit sel d'acide gras audit tensioactif est de 1:39 à 39:1
E) de 1 à 20 % en poids d'un humectant optionnel ; et
F) de 1 à 15 % en poids d'un émollient optionnel.

12. Procédé de production d'une composition de savon liquide stable telle que définie dans la revendication 1, ledit procédé comprenant :
A) la fourniture d'une composition de savon d'acide gras comprenant 10 % à 50 % en poids sur la base en poids de la composition totale d'au moins un sel d'acide gras en C₈ à C₂₂ ;
B) éventuellement, l'augmentation du pH du savon d'acide gras en ajoutant une base appropriée ;
C) la combinaison de la composition de savon d'acide gras obtenue au point (A) ou (B) avec un polymère d'émulsion acrylique réticulé polymérisé à partir de la composition de monomère comprenant ;
- i) un ou plusieurs monomères insaturés contenant un acide carboxylique et des sels de ceux-ci ayant au total 3 à 10 atomes de carbones ;
- ii) un ou plusieurs monomères vinyliques choisis parmi les formules :
-- a) CH₂=CXY
dans laquelle X est H et Y est -COOR, C₆H₄R', -CN, -CONH₂, -Cl, NC₄H₆O, -NH(CH₂)₃COOH, -NHCOCH₃, -CONHC(CH₃)₃, -CO-N(CH₃)₂,
ou X est CH₃ et Y est -COOR, -C₆H₄R', -CN ou -CH=CH₂,
ou X est Cl et Y est Cl, et
R est un groupe alkyle en C₁ à C₁₈ ou hydroxy-alkyle en C₂ à C₁₈,
R' est H ou un groupe alkyle en C₁ à C₁₈ ;
-- b) CH₂=CHOC(O)R¹
dans laquelle R¹ est un groupe alkyle en C₁ à C₁₈; et
-- c) CH₂=CH₂ ou CH₂=CHCN₃ ; et
- iii) un ou plusieurs monomères polyinsaturés ; et
D) la réduction du pH de la composition obtenue au point (C) d'au moins 0,5 à 5 unités de pH inférieures, par l'addition d'un agent acidifiant pour obtenir une composition de savon liquide ayant un pH final de 7,8 à 9,8.

13. Procédé selon la revendication 12, dans lequel ledit monomère contenant un acide carboxylique insaturé est choisi parmi l'acide (méth)acrylique, l'acide itaconique, l'acide fumarique, l'acide crotonique, l'acide maléique, l'acide aconitique, les semi-esters de polyacides avec des alcools en C₁ à C₄, et leurs mélanges

14. Procédé selon la revendication 12, dans lequel ledit polymère d'émulsion acrylique réticulé est polymérisé à partir de l'acide (méth)acrylique et un alkyl-(méth)acrylate en C₁ à C₅.

15. Procédé selon la revendication 12, dans lequel ledit agent acidifiant est choisi parmi un acide organique, un acide inorganique et leurs mélanges.

16. Procédé selon la revendication 12, dans lequel ledit agent acidifiant est choisi parmi l'acide citrique, l'acide acétique, un acide alpha-hydroxy, un acide bêta-hydroxy, l'acide salicylique, l'acide lactique, l'acide glycolique, les acides de fruits naturels, l'acide chlorhydrique, l'acide nitrique, l'acide sulfurique, l'acide sulfamique, l'acide phosphorique et leurs combinaisons.
